# EUROPEAN PATENT SPECIFICATION

(11) **EP 3 052 552 B1**
(45) Date of publication and mention of the grant of the patent: **31.07.2019**
(21) Application number: 14848774.7
(22) Date of filing: 26.09.2014
(51) Int. Cl.: A61F 13/515, A61F 13/84, C08J 3/20, C08J 3/18, C08K 5/04, C08L 101/00, C08J 5/18, C08J 3/215, C08K 5/00

(54) **THERMOPLASTIC ARTICLE WITH THERMAL ACTIVE AGENT**
KUNSTSTOFFARTIKEL MIT EINEM THERMISCHEN WIRKSTOFF
ARTICLE THERMOPLASTIQUE COMPRENANT UN AGENT ACTIF THERMIQUE

(30) Priority: 30.09.2013 US 201361884616 P
(43) Date of publication of application: 10.08.2016
(73) Proprietor: Kimberly-Clark Worldwide, Inc., Neenah, Wisconsin 54956 (US)
(72) Inventor: ZHOU, Peiguang, Neenah, Wisconsin 54956 (US); IWANSKI, David, Gerard, Neenah, Wisconsin 54956 (US); TURCHAN, Gary, Alan, Neenah, Wisconsin 54956 (US); LONG, Andrew, Mark, Neenah, Wisconsin 59456 (US)
(74) Representative: Dehns
(86) International application number: PCT/US2014/057729
(87) International publication number: WO 2015/048450

(56) References cited:
- EP-B1- 0 250 187
- WO-A1-2006/126845
- WO-A2-2008/132618
- WO-A2-2011/077298
- GB-A- 1 431 092
- GB-A- 2 302 651
- US-A- 6 072 100
- US-A1- 2009 291 282
- US-A1- 2009 326 093
- US-A1- 2012 021 026
- US-B1- 6 375 963
- US-B2- 8 147 965

## Description

### BACKGROUND

This disclosure relates to a personal care product containing an extruded, water-soluble, thermoplastic film into which an active agent has been incorporated The active agent has a thermal effect on human skin, either actual or perceived.

Thermal active agents such as menthol and menthol derivatives, and related cooling compounds are used widely in products such as medication, toothpaste, confectionary, cosmetics and pesticides. In recent years, thermal active agents such as polyols (sugar-alcohols), has been used as a cooling agent in children's training pants so that the child is alerted when a urine insult has occurred. It is contemplated that thermal active agents could be used in incontinence and feminine care products as well.

The incorporation of thermal active agents into personal care products such as diapers and pads is not only relatively expensive, but may pose great technical challenges during product manufacture especially if the active agent is volatile in nature. Currently, the thermal active agent (e.g. menthol) is in a powder form (e.g. microencapsulated). The powder may be disposed between two substrates to create a laminate, though acquiring an adhesive bond between the substrates and the powder is challenging. In the alternative, the powder may be formulated into a waxy material and slot-coated onto a product component. However, it is difficult to achieve a uniform slot-coated layer because some of the powder will precipitate from the formulation during the slot-coating process. Further, the formulation has a distinct smell that may be undesirable. Thermal active agents in encapsulated form (e.g. menthol) have at least three disadvantages over crystalline or other non-encapsulated forms. Such disadvantages include: 1) instability due to moisture and temperature sensitivity, 2) three to five times greater manufacturing costs, and 3) sensitive to physical force.

When incorporating a thermal active agent into a thermoplastic article, it is desirable that the: 1) the method of manufacture is cost-effective, and/or 2) the thermoplastic article may easily be incorporated in personal care product, and/or 3) the thermal active agent is quickly released when the thermoplastic article is wetted, and/or 4) the thermal active agent remains stable until it is purposefully placed into a triggering environment.
GB 1431092 discloses a self-lubricating composition for hygiene and medical applications.
GB 2302651 discloses a cold patch. US 2012/0021026 discloses a dissolvable fibrous web structure.
US 6,375,963 discloses a hot-melt extruded film. US 2009/0291282 discloses a water-soluble film.
US 6,072,100 discloses extrudable compositions for the topical application of medicaments.
WO 2006/126845 discloses a method of preparing vacuum - or compression - formable coloured polymeric film.
WO 2011/077298 discloses an absorbent article comprising a signal element.

### SUMMARY

In one aspect of the disclosure is a personal absorbent article which is a diaper or a pant, and which includes an absorbent member sandwiched between a water-impermeable backsheet and a water-permeable liner, wherein the liner has a body-facing surface and an opposite outward-facing surface. A film of the present disclosure is attached to either the outward-facing surface of the liner or a surface of the absorbent member that is adjacent the liner. The film is made from materials that include a water-soluble, polymer having an extrusion temperature of 90 to 150°C; a plasticizer; and one or more active agents in a total amount of 0.1 % to 20% by weight of the article. The active ingredients are dissolvable/dispersible in the plasticizer. The combination of the active agents and plasticizer forms a homogeneous mixture/solution. A homogeneous blend, made from melt-blending the polymer and the homogeneous mixture/solution, has an extrusion temperature of 50 to 125°C.

Other features and aspects of the invention will become apparent by consideration of the detailed description and accompanying drawings.

### BRIEF DESCRIPTION OF THE DRAWINGS

The foregoing and other features and aspects of the present invention and the manner of attaining them will become more apparent, and the invention itself will be better understood by reference to the following description, appended claims and accompanying drawings, where;
- Fig. 1: is a side elevation of one embodiment of a laminate according to the present disclosure;
- Fig. 2: is a side elevation of another embodiment of a laminate according to the present disclosure;
- Fig. 3: is an exploded schematic side-elevation of an exemplary absorbent article;

Repeat use of reference characters in the present specification and drawings is intended to represent same or analogous features or elements of the invention.

### DETAILED DESCRIPTION

It is to be understood by one of ordinary skill in the art that the present discussion is a description of exemplary aspects of the present invention only, and is not intended as limiting the broader aspects of the present invention.

The term "fiber" in the context of the present disclosure generically refers to either meltblown or spunbond fibers as defined herein.

The term "laminate" refers to a material where a film structure is adhesively or non-adhesively bonded to a web such as a nonwoven or tissue material.

The term "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. In the particular case of a coform process, the meltblown fiber stream intersects with one or more material streams that are introduced from a different direction. Thereafter, the meltblown fibers and other optional materials are carried by the high velocity gas stream and are deposited on a collecting surface. The distribution and orientation of the meltblown fibers within the formed web is dependent on the geometry and process conditions. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super-Fine Thermoplastic Fibers" by K. D. Lawrence, R. T. Lukas and J. A. Young; and U.S. Patent No. 3,849,241 to Butin et al. and U.S. Patent No. 5,350,624 to Georger et al.

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblown processes, spunbond processes, air laying processes, wet layering processes and bonded-carded-web processes.

The term "personal care absorbent articles" or "absorbent articles" in the context of this disclosure includes, but is not limited to diapers, diaper pants, training pants, absorbent underpants, incontinence products, and urinary shields; and the like.

The terms "spunbond" and "spunbond fiber" refer to fibers which are formed by extruding filaments of molten thermoplastic material from a plurality of fine, usually circular, capillaries of a spinneret, and then rapidly reducing the diameter of the extruded filaments.

The term "% by weight," "weight %," "wt%" or derivative thereof, when used herein, is to be interpreted as based on the dry weight, unless otherwise specified.

These terms may be defined with additional language in the remaining portions of the specification.

The present disclosure is generally directed to a personal absorbent article as defined in claim 1, comprising an extruded, water-soluble, thermoplastic film into which a thermal active agent has been incorporated. The thermoplastic water-soluble, polymer from which the film is made has an extrusion temperature of 90°C to 150°C. During the film manufacture, a plasticizer is mixed with the polymer, with the plasticizer having one or more thermal active agents dissolved or otherwise dispersed therein. Together, the active agent(s) and the plasticizer form a homogeneous "mixture/solution". The combination of the polymer and the homogeneous mixture/solution form a homogeneous "blend" having an extrusion temperature of 50°C to 125°C.

### Materials

The materials from which the water-soluble, thermoplastic material of the present disclosure is made include a polymer, a plasticizer, and an active agent. Other optional materials that improve the performance, cost, look, feel and/or durability may be added to the thermoplastic material.

**Polymer:** The polymer used in the present disclosure is water soluble or water dispersible. Such materials include polyvinyl alcohol (PVOH), polyethylene oxide (PEO), polyethylene glycol (PEG), polyacylate (acid), polyacylamide, polyester, or a combination of one or more of these polymers. The polymer has an extrusion temperature of 90°C to 150°C.

One desirable polymer is a highly amorphous vinyl alcohol polymer, sold as "NICHIGO G-POLYMER," available from Soarus L.L.C., Arlington Heights, Illinois. This particular polymer has a molecular weight of 10,000 to 50,000, and a relatively low crystallinity of 5 to 25%.

In one aspect, a copolymer such as ethylene vinyl acetate (EVA) may be combined with the base polymer. It is contemplated that the film of the present disclosure may include up to 30% by weight EVA.

**Plasticizer:** The plasticizer functions to a) reduce the polymer coextrusion temperature and b) act as a carrier to distribute the molecules of the thermal active agent into the polymer matrix. The plasticizer enables materials that are incompatible with the polymer to be blended therewith. A plasticizer is able to receive the thermal active agent within its internal structure, either by dissolution or dispersion, to form a homogeneous mixture/solution.

All suitable plasticizers uniformly dissolve the thermal active agent of choice, described herein. Suitable plasticizers include glycerin, PEG-400, PEG-800, PEG-1000, and low molecular weight polypropylene oxide/polyethylene oxide-based copolymers. One desirable plasticizer is a liquid POLYGYKOL - 400, available from Clariant KS.

Typically, the plasticizer constitutes from about 0 wt. % to about 40 wt. %, in some embodiments from about 5 wt. % to about 30 wt. %, and in some embodiments, from about 10 wt. % to about 20 wt. % of the homogenous blend.

**Active Agent:** In one aspect, the active agent(s) stimulates human sensory receptors to give the impression that a change in temperature has occurred upon contact with the skin. In another aspect, the active agent(s) actually change the temperature of the skin. In either aspect, the temperature change may be described as either cooling or warming, depending on the active agent.

The active agent(s) is mixed with the plasticizer as described herein. Suitable active agents include menthol, menthol derivatives, xylitol, capsaicin, polyols (sugar alcohol), urea, self-heating zeolite or alkali metal-compounds, magnesium-based compounds such as magnesium chloride and magnesium sulfate. Some of these active agents are volatile materials (e.g. menthol or menthol derivatives, in particular, menthol with a flash point of 93°C).

The active agent(s) is present in a total amount of 0.1% to 20% by weight of the film, or a total amount of 1% to 20% by weight of the film. In another aspect, the active agent(s) is present in a total amount of 2% to 10% by weight of the film.

**Optional Materials:** Besides the components noted above, still other additives may also be incorporated into the composition, such as fragrances, melt stabilizers, dispersion aids (e.g., surfactants), processing stabilizers, heat stabilizers, light stabilizers, UV stabilizers, antioxidants, heat aging stabilizers, whitening agents, antiblocking agents, antistatic agents, bonding agents, lubricants, etc.

In one aspect of the present disclosure, the extruded water-soluble film includes up to 50% thermoplastic starch by weight. The thermoplastic starch acts as a filler to reduce the overall cost of the extruded film. The extruded film may contain as much as 30% thermoplastic starch. One desirable water-soluble thermoplastic starch is a cellulose based starch obtained from various plant sources, hemicelluloses, modified cellulose (hydroxylalkyl cellulose, cellulose ethers, cellulose esters, etc.), and so forth. When a thermoplastic starch is employed, the amount of such additional material may range from about 1 wt. % to about 50 wt. %, in some embodiments from about 5 wt. % to about 40 wt. %, and in some embodiments, from about 10 wt. % to about 30 wt. % of the homogeneous blend.

Dispersion aids may also be employed to help create a uniform dispersion of the active agent/plasticizer. When employed, the dispersion aid(s) typically constitute from about 0.01 wt. % to about 10 wt. %, in some embodiments from about 0.1 wt. % to about 5 wt. %, and in some embodiments, from about 0.5 wt. % to about 4 wt. % of the homogeneous blend.

The composition may also contain a preservative or preservative system to inhibit the growth of microorganisms over an extended period of time. Suitable preservatives may include, for instance, alkanols, disodium EDTA (ethylenediamine tetraacetate), EDTA salts, EDTA fatty acid conjugates, isothiazolinone, benzoic esters (parabens) (e.g., methylparaben, propylparaben, butylparaben, ethylparaben, isopropylparaben, isobutylparaben, benzylparaben, sodium methylparaben, and sodium propylparaben), benzoic acid, propylene glycols, sorbates, urea derivatives (e.g., diazolindinyl urea), and so forth. Other suitable preservatives include those sold by Sutton Labs, such as "Germall 115" (amidazoiidinyl urea), "Germall II" (diazolidinyl urea), and "Germall Plus" (diazolidinyl urea and iodopropynyl butylcarbonate). Another suitable preservative is Kathon CG.RTM., which is a mixture of methylchloroisothiazolinone and methylisothiazoiinone available from Rohm & Haas; Mackstat H 66 (available from McIntyre Group, Chicago, III.). Still another suitable preservative system is a combination of 56% propylene glycol, 30% diazolidinyl urea, 11% methylparaben, and 3% propylparaben available under the name GERMABEN.RTM. II from International Specialty Products of Wayne, N.J.

To provide additional benefits to consumers, optional ingredients may be added to either the homogeneous mixture/solution or homogeneous blend. For instance, classes of optional ingredients that may be used include, but are not limited to: antioxidants (for product integrity); astringents-cosmetic (for inducing a tingling sensation on skin); colorants (for imparting color to the product); deodorants (for reducing or eliminating unpleasant odors and for protecting against the formation of malodor on body surfaces); fragrances (for consumer appeal); skin conditioning agents; skin protectants (a product which protects injured or exposed skin or mucous membrane surface from harmful or annoying stimuli); and antimicrobials.

It is desirable that the films of the present disclosure are formed without the use of solvents, particularly organic solvents, such as organic alcohols (e.g., ethanol). This can limit the evaporation of any volatile active agent that may be incorporated into the article.

### Method of Manufacture

In one aspect of the disclosure, a method of making an extruded film includes the following steps. First, prepare a homogeneous mixture/solution of a thermoplastic plasticizer and one or more thermal active agents. Second, form a homogenous blend by combining the homogeneous mixture/solution and a polymer. In one desired embodiment, the polymer is an amorphous, water-soluble vinyl alcohol as described herein. The resulting homogeneous blend includes one or more thermal active agents. Third, extrude the homogeneous blend to form a film.

The homogeneous blend, made by combining the polymer and the homogeneous mixture/solution, has an extrusion temperature of 50°C to 125°C, or possibly, ranging from 90°C to 125°C. This relatively low extrusion temperature profile prevents the thermal active agent from phase separating and results in a homogeneous blend of at least one polymer and thermal active agent(s) at the extrusion stage. In some aspects, the macrophage separation of a crystallizing active agent (e.g. menthol or its derivatives) is prevented.

**Extrusion Method:** The composition of the present disclosure is formed by processing the components together in a melt-blending device (e.g., extruder). The mechanical shear and heat provided by the melt-blending device allow the components to be blended together in a highly efficient manner without the use of a solvent. Batch and/or continuous melt-blending techniques may be employed in the present disclosure. For example, a mixer/kneader, Banbury mixer, Farrel continuous mixer, single-screw extruder, twin-screw extruder, roll mill, etc., may be utilized. One particularly suitable melt-blending device is a twin-screw extruder (e.g., PRISM USALAB x16, available from Thermo Electric Co., Inc., New Jersey).

Raw materials are supplied to the melt-blending device in two phases. For example, the thermal active agent is dissolved or otherwise dispersed in the plasticizer to form the homogenous mixture/solution, thereafter, this mixture is injected into the extruder downstream from the polymer and other optional ingredients as described herein, e.g. starch.

The polymer and the homogeneous mixture/solution together form a homogeneous blend. These components may be blended at a shear/pressure and temperature sufficient to ensure adequate mixing (e.g., at or above the melting point of the polymer), but without adversely impacting the physical properties of the thermal active agent. For example, melt-blending typically occurs at a temperature of from about 50°C to about 150°C., in some embodiments, from about 70°C to about 125°C, and in some embodiments from about 80°C to about 99°C. These relatively low processing temperatures prevent any volatilization of the active agent as well as reduce any thermally induced chemical reaction.

The following extruder setup is desirable:
- Extruder extrusion Temperature Range: 90-125°C
- Temperature setting profile for 125°C extrusion temperature:
   - Feed section 100-110°C
   - Compression section 110-120°C
   - Metering section 120-125°C
   - Die 120-130°C
- Extrusion feed rate: 2.00 - 4.00 pounds/minute
- Screw speed: 100-250 rpm

Once formed, the homogeneous blend of the present disclosure is used to create a film.

Films: The homogeneous blend is formed into a film, either alone or in conjunction with an additional film-forming material. The film is used in the personal absorbent articles of the present invention. The film may have a mono- or multi-layer configuration. Desirably, the film thickness ranges from 0.025 mm to 0.25 mm. Any known technique may be used to form a film from the compounded material, such as extrusion coating, coextrusion of the layers, or any conventional layering process.

Regardless of how the film is formed, it may be optionally oriented in one or more directions to further improve film uniformity and reduce thickness. For example, the film may be immediately reheated to a temperature below the melting point of one or more polymers in the film, but high enough to enable the composition to be drawn or stretched. In the case of sequential orientation, the "softened" film is drawn by rolls rotating at different speeds or rates of rotation such that the sheet is stretched to the desired draw ratio in the longitudinal direction (machine direction).

The multi-layer film may contain from two (2) to eight (8) layers, and in some embodiments, from three (3) to five (5) layers. In one example, the multi-layer film has one base layer and one skin layer. The base layer and/or skin layer may contain the thermal active agent(s). The ratio between the layers may range from 20 to 1.

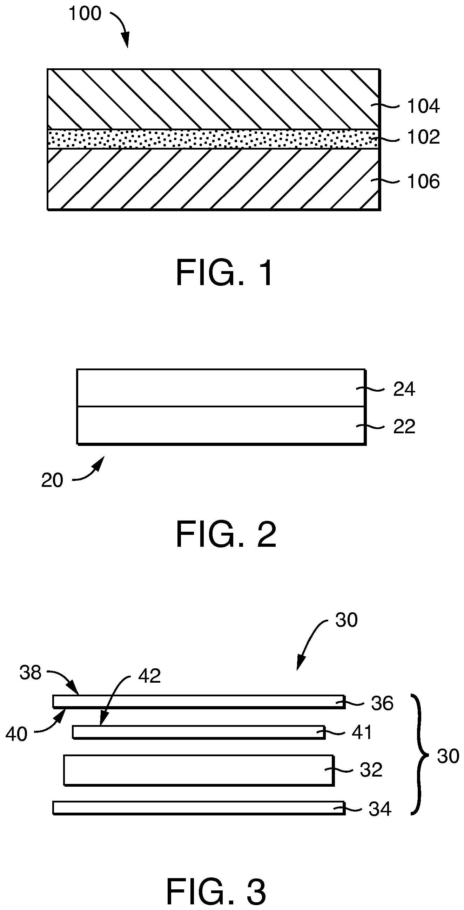

Another example as shown in FIG. 1, is a three-layered film 100 having a core layer 102 containing thermal active agents. The outer skin layers 104 and 106 may be polyolefin (e.g. EVA). The ratio between the layers may range from 60% to 40% of the core layer and from 90% to 10% of the two combined skin layers. For instance, the core layer may be up to about 30%, up to about 40%, up to about 50%, up to about 60%, or up to about 70% of the total thickness of the multi-layer film. Each skin layer may be up to about 15%, or up to about 25%, or up to about 35% of the total thickness of the multi-layer film. The skin layer may be of different thicknesses with respect to one another.

The film, either mono- or multi-layered, may be wound and stored on a take-up roll. Various additional potential processing and/or finishing steps known in the art, such as slitting, treating, aperturing, printing graphics, or lamination of the film with other layers (e.g., nonwoven web materials), may be performed.

In one aspect, the extruded water-soluble film has a basis weight of 5 gsm to 500 gsm. In another aspect, the water-soluble film has a basis weight of 20 gsm to 200 gsm.

In one aspect, the extruded water-soluble film has a tensile strength of 0.5 MPa to 50 MPa according to the Tensile Test of the present disclosure. In another aspect, the film has a tensile strength of 1 MPa to 25 MPa according to the same test.

In one aspect, the extruded water-soluble film has a water dissolution speed from 5 seconds to 30 minutes as determined by the Dissolution Test of the present disclosure. In another aspect, the extruded water-soluble article film has a water dissolution speed of 30 seconds to 5 minutes as determined by the same test.

In one aspect, the extruded water-soluble film demonstrates an elongation of 5% to 500% according to the Tensile Test of the present disclosure. In another aspect, the film demonstrates an elongation of 10% to 100% according to the same test.

Articles (not claimed): The homogeneous blend of the present invention may also be used to form other types of articles. In one aspect, the extruded water-soluble article is a rod having a circular- or elliptical-shaped extrusion profile. In another aspect, the extruded water-soluble article is a rod having the geometric extrusion profile of a polygon with three to ten sides (e.g. a triangle to a decagon). The rod may be cut into pellets for later processing.

Referring to **Fig. 2****,** a laminate 20 may be formed by extruding the homogeneous blend 24 onto a carrier substrate 22, forming a bond therebetween. The carrier substrate 22 may be a nonwoven material, woven material, cellulose web or the like. Such a laminate may be used as a sheet or as a component of an absorbent article such as a bandage, diaper, pad, medical drape, and pants as noted herein.

In one aspect, the laminate 20 may be used as a wipe that is sold to customers in a dry state, and wetted by the customer. The customer may be the end consumer. This provides at least the advantage of reduced shipping costs.

### Applications

**Absorbent Articles:** The film described above is particularly suitable for use in an absorbent article. An "absorbent article" generally refers to any article capable of absorbing water or other fluids. Examples of some absorbent articles includepersonal care absorbent articles, such as diapers, training pants, absorbent underpants and so forth. Several examples of such absorbent articles are described in U.S. Patent Nos. 5,649,916 to DiPalma, et al.; 6,110,158 to Kielpikowski; 6,663,611 to Blaney, et al.. Still other suitable articles are described in U.S. Patent Application Publication No. 2004/0060112 A1 to Fell et al., as well as U.S. Patent Nos. 4,886,512 to Damico et al.; 5,558,659 to Sherrod et al.; 6,888,044 to Fell et al.; and 6,511,465 to Freiburger et al. Materials and processes suitable for forming such absorbent articles are well known to those skilled in the art.

The present invention may be better understood with reference to the examples presented herein.

**Exemplary Absorbent Article:** Referring to Fig. 3, in one aspect of the disclosure, a personal absorbent article 30 includes an absorbent member 32 sandwiched between a water-impermeable backsheet 34 and a water-permeable liner 36, wherein liner 36 has a body-facing surface 38 and an opposite outward-facing surface 40. A film 41 of the present disclosure is attached to either the outward-facing surface 40 of the liner or a surface 42 of the absorbent member 32 that is positioned adjacent to liner 36. Desirably, film 41 is in direct contact with liner 36. Should a multi-layer film be used for film 41, the layer containing the largest amount of active agent is adjacent liner 36 so that the active agent can more easily leach through the liner 36 to contact the wearer's body.

As described, film 41 is made from materials that include a water-soluble, polymer that has an extrusion temperature of 90 to 150°C; a plasticizer; and one or more thermal active agents in a total amount of 0.1 % to 20% by weight of the film.

### Experimental Data

A twin-screw extruder (PRISM USALAB x16, available from Thermo Electric Co., Inc.) was used to make co-extruded film samples that contain menthol. The extruder specifications were as follows:
- 16 mm diameter screw
- L/D=40 (L=640 mm)
- 10 heating zones + die
- Maximum velocity = 1000 rpm
- Maximum pressure = 100bar
- Maximum torque = 24 mN

The following extruder set-up was used to manufacture the experimental film:
- Flat slit die width: 152.40mm (6")
- Flat slit die height (controls film thickness): 0.127mm (0.010")

The coextruded film included menthol (99% purity, crystal form, from Sigma-Aldrich Corp., St. Louis, MO). 100 g of the menthol was mixed with 400 g of Polyglykol-400 liquid (from Clariant, Charlotte, NC.). To make a homogeneous solution of the mixture, the mixture was heated in an oven at 60°C to completely dissolve the menthol. (As desired, the resulting homogeneous menthol/polyglykol solution failed to phase separate after overnight storage in an oven set at 25°C.)

The extruder feed zone was heated to 110°C, the following extruder zones 2-9 were heated to 125°C, and the die was heated to 130°C.

An amorphous polymer, namely the G-Polymer from Nippon-Goshie as described herein; and a thermoplastic starch, Thermalplastic Starch Glucose-800 from Chemstar Products Company, Minneapolis, MN, were fed into the extruder feed section at 1.0 g/min. The homogeneous menthol/polyglykol solution was also pumped into the extruder feed section at a rate of 1.0 g/min. A film of 2-5 mils (51-127µm) thickness was extruded at a speed of 150-200 RPM.

### Cooling Sensation Test:

The cooling sensation provided by the menthol film was subjectively determined as follows. First, a film containing 2% menthol (as prepared above) was dipped into water having a temperature of about 23°C and then attached to the forearm of a human test subject. The lab conditions were at approximately 50% relative humidity, 23°C.

The test subject reported feeling a cooling sensation within 2 minutes after placement of the film on the forearm. The cooling sensation was reported to last from 15-30 minutes. Control specimens containing no menthol did not provide any cooling sensation.

### Dissolution Test:

The dissolution test is a procedure for determining an estimation of the dissolution time of a polymer film in deionized water or saline. ASTM Standard Test Method D5226-98 "Standard Practice for Dissolving Polymer Materials" was used with any test variances noted below. This test is used to 1) compare the relative dissolution times of different types of polymers, and 2) observe the effects of additives and/or other active agents blended with the polymers. Although times are recorded for each test code, this test is not quantitative because one must visually determine the end point of the film dissolution.
1. Specimen Preparation
   a. Sample polymer films were cut into 2.54 cm x 15.24 cm (1 inch x 6 inch) strips using a die and press.
   b. Three specimens were cut for each film sample.
2. Experimental Set up and Procedure
   a. Deionized water or saline is placed in a 1 L glass beaker and heated to 37°C in a hot water bath to facilitate less down time between sample tests.
   b. The mass of each strip is measured using a standard balance.
   c. It is desirable to have the mass of film to mass of medium ratio be 0.5%. Therefore, based on the measured mass, the appropriate mass of medium is calculated.
   d. After the medium is conditioned to ambient temperature, the appropriate mass-of-medium is obtained using the balance. The medium is placed in a 250 mL glass beaker with a 1.5 inch (3.8cm) magnetic stir rod.
   e. This beaker is then placed on a VWR Hotplate/Stirrer with a feedback thermometer. The temperature of the medium is then checked to ensure the medium was at 37°C. The stir rate is set to 100 rpms.
   f. The film is then folded in half (to prevent film adherence to the sides of the beaker) and placed on the surface of the water. At this point, the timer is started.
   g. The film in solution is then closely monitored to observe the dissolution process of the
   h. film and to determine the end point of dissolution.
   i. The end point is determined primarily by the size of the intact film left in solution.
      i. 90% dissolution is set as the standard for the end point. When most of the large particles are dissolved, the end point is recorded.
      ii. The goal is to go from 6 in² (39cm²) to around 0.6 in² (3.9cm²) surface area. If the film curls into a ball, this means a radius of about 6.4 mm.
      iii. Haziness of the water or saline solution is a secondary factor.
         1. Many of the films are an off white or black, and upon dissolution, cause the solution to become cloudy. However, a cloudy solution aids in determining the end point.
   j. Accuracy check
      i. Because the end point is assessed visually, a check is performed to determine accuracy. Once an end point is determined, the film solution is monitored for an additional two times the length of the trial to ensure the film does not significantly continue to dissolve. For example, if the end point for a film was determined to be 45 seconds, then the film is monitored for an additional 90 seconds to ensure further dissolution does not occur and to help increase the accuracy and repeatability of the test.
3. Data Analysis
   a. Dissolution time results are recorded for each sample and averages are calculated for a statistical comparison of different polymer blends.

The menthol film, as prepared above, had a dissolution speed of about 5 seconds.

**Tensile Test:** Prior to testing, film specimens are initially conditioned at 75°F (24°C) and 50% relative humidity for 24 hours. Thereafter, tensile strength values are determined in accordance with ASTM Standard D882-10.

A constant-rate-of-extension type of tensile tester is employed such as an MTS Synergie 200 Mechanical Tester (MTS System Co., MN, USA). An appropriate load cell is selected so that the tested value fell within the range of 10-90% of the full scale load.

Specimen dimensions are 101.6mm x 9.525 mm (4" x 3/8") in accordance with ASTM Standard Test Method D6287 "Standard Practice for Cutting Film and Sheeting Test Specimens".

The specimens were held between rubberized grips at a gauge length of 50.8 mm (2 inch). Specimens were stretched at a crosshead speed of about 508 mm/min (20 inch/min) until failure occurred. At least sixteen samples were tested, at least eight by applying the test load in the machine-direction of the specimen, and at least eight samples were tested by applying the test load in the cross direction of the specimen. The modulus of elasticity, peak load, peak stress, elongation (percent strain at break), and energy per volume at break (total area under the stress-strain curve) may be obtained from the results.

## Claims

1. A personal absorbent article which is a diaper or a pant, said article comprising:
an absorbent member sandwiched between a water-impermeable backsheet and a water-permeable liner, wherein the liner has a body-facing surface and an opposite outward-facing surface; and
a film attached to either the outward-facing surface of the liner or a surface of the absorbent member that is adjacent the liner;
wherein the film comprises:
a water-soluble polymer having an extrusion temperature of 90 to 150°C;
a plasticizer; and
one or more thermal active agents in a total amount of 0.1 % to 20% by weight of the film;
wherein the one or more thermal active ingredients are dissolvable in the plasticizer;
wherein the combination of the one or more thermal active agents and the plasticizer is a homogeneous mixture/solution; and
wherein a homogeneous blend comprising the polymer and the homogeneous mixture/solution has an extrusion temperature of 50 to 125°C.

2. The personal absorbent article of claim 1, wherein the polymer is selected from the group consisting of polyvinyl alcohol (PVOH), polyethylene oxide (PEO), polyethylene glycol (PEG), polyacylate (acid), polyacylamide, polyester, thermoplastic starch, polyolefin copolymer; and a combination thereof.

3. The personal absorbent article of claims 1 and 2, wherein the plasticizer is selected from glycerin, PEG- 400, PEG- 800, PEG-1000, and low molecular weight polypropylene oxide/polyethylene oxide-based copolymers.

4. The personal absorbent article of claims 1 and 2, wherein the polymer is an amorphous vinyl alcohol matrix.

5. The personal absorbent article of claims 1 and 2, wherein the homogeneous blend comprising the polymer and the homogeneous mixture/solution has an extrusion temperature of 90 to 125°C.

6. The personal absorbent article of claims 1 and 2, wherein the one or more thermal active agents are in a total amount of 2% to 10% by weight of the film.

7. The personal absorbent article of claims 1 and 2, wherein the film further comprises up to 50% thermoplastic starch by weight.

8. The personal absorbent article of claims 1 and 2, wherein the film further comprises up to 30% by weight of ethylene vinyl acetate.

9. The personal absorbent article of claim 1, wherein the film is a mono-layer or multi-layer film.

10. The personal absorbent article of claim 9, wherein the film has a water dissolution speed from 5 seconds to 30 minutes as determined by the Dissolution Test of the present disclosure; and/or
wherein the film has a basis weight of 5 gsm to 500 gsm; and/or
wherein the film has an elongation of 5% to 500% according to the Tensile Test of the present disclosure.

11. The personal absorbent article of claims 1 and 2 wherein the one or more thermal active agents are menthol, a menthol derivative thereof, or a combination thereof.

12. The personal absorbent article of claims 1 and 2 wherein the one or more thermal active agents are configured to either stimulate human sensory receptors or change the temperature of the skin upon contact therewith; and/or
wherein the one or more thermal active agents comprise a temperature agent selected from menthol, menthol derivatives, xylitol, capsaicin, polyols, urea, self-heating zeolite or alkali metal-compounds, magnesium chloride, magnesium sulfate and combinations thereof.

## Patentansprüche

1. Saugfähiger Körperpflegeartikel, der eine Windel oder eine Hose ist, der Artikel umfassend
ein saugfähiges Element, das sich sandwichartig zwischen einer wasserundurchlässigen Außenschicht und einer wasserdurchlässigen Auskleidung befindet, wobei die Auskleidung eine zum Körper weisende Fläche und eine gegenüberliegende, nach außen weisende Fläche hat; und
eine Folie, die entweder an der nach außen weisenden Fläche der Auskleidung oder einer Fläche des saugfähigen Elements angebracht ist, die sich neben der Auskleidung befindet;
wobei die Folie Folgendes umfasst:
ein wasserlösliches Polymer mit einer Extrusionstemperatur von 90 bis 150 °C;
einen Weichmacher; und
ein oder mehrere thermische Wirkstoffe in einer Gesamtmenge von 0,1 bis 20 Gewichts-% der Folie;
wobei die ein oder mehreren thermischen Wirkstoffe im Weichmacher löslich sind;
wobei die Kombination der ein oder mehreren thermischen Wirkstoffe und des Weichmachers eine homogene Mischung/Lösung ist; und
wobei ein homogenes Gemisch, das das Polymer und die homogene Mischung/Lösung umfasst, eine Extrusionstemperatur von 50 bis 125 °C hat.

2. Saugfähiger Körperpflegeartikel nach Anspruch 1, wobei das Polymer aus der Gruppe ausgewählt wird, die aus Polyvinylalkohol (PVOH), Polyethylenoxid (PEO), Polyethylenglycol (PEG), Polyacylat (Säure), Polyacylamid, Polyester, thermoplastischer Stärke, Polyolefin-Copolymer und einer Kombination davon besteht.

3. Saugfähiger Körperpflegeartikel nach Ansprüchen 1 und 2, wobei der Weichmacher aus Glycerin, PEG-400, PEG-800, PEG-1000 und polypropylenoxid-/polyethylenoxid-basierten Copolymeren mit geringem Molekulargewicht ausgewählt wird.

4. Saugfähiger Körperpflegeartikel nach Ansprüchen 1 und 2, wobei das Polymer eine amorphe Vinylalkoholmatrix ist.

5. Saugfähiger Körperpflegeartikel nach Ansprüchen 1 und 2, wobei das homogene Gemisch, das das Polymer und die homogene Mischung/Lösung umfasst, eine Extrusionstemperatur von 90 bis 125 °C hat.

6. Saugfähiger Körperpflegeartikel nach Ansprüchen 1 und 2, wobei die ein oder mehreren thermischen Wirkstoffe in einer Gesamtmenge von 2 bis 10 Gewichts-% der Folie vorhanden sind.

7. Saugfähiger Körperpflegeartikel nach Ansprüchen 1 und 2, wobei die Folie ferner bis zu 50 Gewichts-% thermoplastische Stärke umfasst.

8. Saugfähiger Körperpflegeartikel nach Ansprüchen 1 und 2, wobei die Folie ferner bis zu 30 Gewichts-% Ethylenvinylacetat umfasst.

9. Saugfähiger Körperpflegeartikel nach Anspruch 1, wobei die Folie eine einlagige oder mehrlagige Folie ist.

10. Saugfähiger Körperpflegeartikel nach Anspruch 9, wobei die Folie eine Wasserauflösungsgeschwindigkeit von 5 Sekunden bis 30 Minuten, wie durch die Auflösungsprüfung der vorliegenden Offenbarung ermittelt, hat, und/oder
wobei die Folie ein Flächengewicht von 5 g/m² bis 500 g/m² hat; und/oder
wobei die Folie eine Dehnung von 5 % bis 500 % gemäß der Zugprüfung der vorliegenden Offenbarung hat.

11. Saugfähiger Körperpflegeartikel nach Ansprüchen 1 und 2, wobei die ein oder mehreren thermischen Wirkstoffe Menthol, ein Mentholderivat davon oder eine Kombination davon sind.

12. Saugfähiger Körperpflegeartikel nach Ansprüchen 1 und 2, wobei die ein oder mehreren thermischen Wirkstoffe so konfiguriert sind, dass sie entweder die menschlichen sensorischen Rezeptoren stimulieren oder die Temperatur der Haut nach Kontakt damit ändern; und/oder
wobei die ein oder mehreren thermischen Wirkstoffe einen Temperaturwirkstoff umfassen, der aus Menthol, Mentholderivaten, Xylitol, Capsaicin, Polyolen, Urea, selbsterhitzenden Zeolit- oder Alkali-Metall-Verbindungen, Magnesiumchlorid, Magnesiumsulfat oder Kombinationen davon ausgewählt wird.

## Revendications

1. Article absorbant personnel qui est une couche ou une culotte, ledit article comprenant
un élément absorbant intercalé entre une feuille de renfort imperméable à l'eau et une doublure perméable à l'eau, dans lequel la doublure a une surface tournée vers le corps et une surface opposée tournée vers l'extérieur ; et
un film attaché soit à la surface tournée vers l'extérieur de la doublure, soit à une surface de l'élément absorbant qui est adjacente à la doublure ;
dans lequel le film comprend :
un polymère hydrosoluble ayant une température d'extrusion de 90 à 150 °C ;
un plastifiant ; et
un ou plusieurs agents actifs thermiques dans une quantité totale de 0,1 % à 20 % en poids du film ;
dans lequel les un ou plusieurs principes actifs thermiques sont solubles dans le plastifiant ;
dans lequel la combinaison des un ou plusieurs agents actifs thermiques et du plastifiant sont un mélange/une solution homogène ; et
dans lequel un alliage homogène comprenant le polymère et le mélange/la solution homogène a une température d'extrusion de 50 à 125 °C.

2. Article absorbant personnel selon la revendication 1, dans lequel le polymère est choisi dans le groupe constitué par l'alcool polyvinylique (PVOH), l'oxyde de polyéthylène (PEO), le polyéthylène glycol (PEG), le polyacylate (acide), le polyacylamide, le polyester, l'amidon thermoplastique, un copolymère de polyoléfine ; et une combinaison de ceux-ci.

3. Article absorbant personnel selon les revendications 1 et 2, dans lequel le plastifiant est choisi parmi la glycérine, le PEG-400, le PEG-800, le PEG-1000 et des copolymères à base d'oxyde de polypropylène/d'oxyde de polyéthylène de faible poids moléculaire.

4. Article absorbant personnel selon les revendications 1 et 2, dans lequel le polymère est une matrice d'alcool vinylique amorphe.

5. Article absorbant personnel selon les revendications 1 et 2, dans lequel l'alliage homogène comprend le polymère et le mélange/la solution homogène a une température d'extrusion de 90 à 125 °C.

6. Article absorbant personnel selon les revendications 1 et 2, dans lequel les un ou plusieurs agents actifs thermiques sont dans une quantité totale de 2 % à 10 % en poids du film.

7. Article absorbant personnel selon les revendications 1 et 2, dans lequel le film comprend en outre jusqu'à 50 % en poids d'amidon thermoplastique.

8. Article absorbant personnel selon les revendications 1 et 2, dans lequel le film comprend en outre jusqu'à 30 % en poids d'acétate de vinyle-éthylène.

9. Article absorbant personnel selon la revendication 1, dans lequel le film est un film monocouche ou multicouche.

10. Article absorbant personnel selon la revendication 9, dans lequel le film a une vitesse de dissolution dans l'eau de 5 secondes à 30 minutes, telle que déterminée par l'essai de dissolution de la présente divulgation ; et/ou
dans lequel le film a un poids de base de 5 g/m2 à 500 g/m2 ; et/ou
dans lequel le film a un allongement de 5 % à 500 % selon l'essai de traction de la présente divulgation.

11. Article absorbant personnel selon les revendications 1 et 2, dans lequel les un ou plusieurs agents actifs thermiques sont le menthol, un dérivé du menthol ou une combinaison de ceux-ci.

12. Article absorbant personnel selon les revendications 1 et 2, dans lequel les un ou plusieurs agents actifs thermiques sont conçus pour soit stimuler les récepteurs sensoriels humains, soit modifier la température de la peau à son contact ; et/ou
dans lequel les un ou plusieurs agents actifs thermiques comprennent un agent de température choisi parmi le menthol, les dérivés du menthol, le xylitol, la capsaïcine, les polyols, l'urée, la zéolite ou les composés de métaux alcalins auto-chauffants, le chlorure de magnésium, le sulfate de magnésium et des combinaisons de ceux-ci.
